# EUROPEAN PATENT APPLICATION

(11) **EP 4 548 915 A1**
(43) Date of publication of application: **07.05.2025**
(21) Application number: 24209296.3
(22) Date of filing: 28.10.2024
(51) Int. Cl.: A61K 9/50

(54) **METHOD OF MANUFACTURING SOLID PHARMACEUTICAL PREPARATION COATED WITH SOLVENT-FREE COATING MATERIAL**

(30) Priority: 31.10.2023 JP 2023186844; 20.11.2023 JP 2023196374
(71) Applicant: Shin-Etsu Chemical Co., Ltd., Chiyoda-ku, Tokyo 1000005 (JP)
(72) Inventor: SAKAI, Hidetoshi, KANAGAWA, 2400005 (JP); HOSHINO, Takafumi, KANAGAWA, 2400005 (JP)
(74) Representative: Cabinet Nony

(57) **Abstract**

Provided is a method of manufacturing a solid pharmaceutical preparation which enables the coating using spraying of powder, containing HPMCAS, alone and requiring only a single layering process. The method of manufacturing a solid pharmaceutical preparation coated with a solvent-free coating material essentially includes the steps of: mixing a powdered hydroxypropylmethylcellulose acetate succinate, a powdered plasticizer, and a powdered wetting agent in advance, as a preliminary mixing step, to prepare a precursor mixture; mixing the precursor mixture and a solid pharmaceutical preparation, as a mixing step, to produce a mixture product; and heating the mixture product, as a heating step, to obtain a coated solid pharmaceutical preparation.

## Description

### BACKGROUND OF THE INVENTION

### Field of the invention

This invention relates to a method of manufacturing a solid pharmaceutical preparation coated with a solvent-free coating material.

### Background art

Coating materials for solid pharmaceutical preparations are widely used for various purposes including, for example, protecting acid-sensitive medicines from stomach acid, achieving pH-dependent dissolution, and masking bitterness.

Examples of coating agents in actual use include cellulose type coating agents such as methylcellulose (MC), hydroxypropylmethylcellulose (HPMC), cellulose acetate phthalate (CAP), cellulose acetate trimellitate (CAT), hydroxypropylmethylcellulose phthalate (HPMCP), hydroxypropylmethylcellulose acetate succinate (HPMCAS) and carboxymethylethylcellulose (CMEC); vinyl type coating agents such as polyvinyl alcohol (PVA), and polyvinyl alcohol acetate phthalate (PVAP); and acrylic type coating agents such as a copolymer of methacrylic acid and ethyl acetate. These coating agents can be dissolved in organic solvents for use as a coating material or as an aqueous latex or an aqueous dispersion. Since organic solvent or water is inevitably used as a solvent in these methods, it must take a long time to spray these coating solutions and dry them. Moreover, some drugs are sensitive to contact with water or organic solvents. Therefore, development of a coating method in which no solvent is used has been desired.

As an example of coating method using no solvent, JP-A-H10-81620 proposes an enteric coating using a polymeric powder such as HPMCAS for coating agent while continuously spraying, onto a solid medicine, a mixture of a plasticizer and a liquid substance which has a contact angle of 10 degrees or less against the polymeric coating agent.

In addition, as an example of carrying out a coating using powder ingredients alone, JP-A-2021-84873 proposes a coating method including three steps of: mixing fine particles, core particles, and wax and layering the fine particles and the wax on the core particles; mixing the laminated particles with a polymer, such as HPMCAS, and coating the laminated particle with the polymer; and curing the polymer-coated particles.

### SUMMARY OF THE INVENTION

However, according to the method of JP-A-H10-81620, atomization of liquid ingredients and feeding of solid ingredients need to be separately performed, and it makes the operation of this technique complicated. Furthermore, in the method of JP-A-2021-84873, since adhesion of the polymer to the core particles is poor, the method needs two-stages of coating process; Firstly, the core particles are coated with wax, and then, the polymer is added and adhered by a binding capability of the wax. Therefore, a long operation time and labor are required.

The present invention has been made in view of the above-mentioned circumstances, and it is an object of the present invention to provide a method of manufacturing a solid pharmaceutical preparation, which enables the coating using spraying of a HPMCAScontaining powder alone and requiring only a single layering process.

The inventors of the present invention diligently conducted studies to solve the aforementioned problems, and completed the invention by finding that blending a wetting agent and a plasticizer as powders with HPMCAS significantly improves adherability of HPMCAS to core particles, and that enables the coating using spraying of the powder alone and requiring only a single layering process.

The present invention provides a method of manufacturing a solid pharmaceutical preparation as defined by the following clauses <1> to <9>.
<1> A method of manufacturing a solid pharmaceutical preparation coated with a solvent-free coating material comprising the steps of:
   mixing a powdered hydroxypropylmethylcellulose acetate succinate, a powdered plasticizer, and a powdered wetting agent in advance, as a preliminary mixing step, to prepare a precursor mixture;
   mixing the precursor mixture and a solid pharmaceutical preparation, as a mixing step, to produce a mixture product; and
   heating the mixture product, as a heating step, to obtain a coated solid pharmaceutical preparation.
<2> The method according to <1>, wherein the mixing step is performed while heating.
<3> The method according to <1> or <2>, wherein the mixing step comprises continually spraying the precursor mixture onto the solid pharmaceutical preparation to produce a mixture product.
<4> The method according to any one of <1> to <3>, wherein the powdered hydroxypropylmethylcellulose acetate succinate has a volume-based average particle diameter of 1 to 10 µm, wherein the volume-based average particle diameter is determined by dry laser diffractometry.
<5> The method according to any one of <1> to <4>, wherein the powdered plasticizer has a melting point of 50 to 69°C.
<6> The method according to any one of <1> to <5>, wherein the powdered plasticizer is at least one material selected from polyethylene glycol and stearic acid.
<7> The method according to any one of <1> to <6>, wherein the powdered wetting agent has a melting point at least 8°C lower than the melting point of the powdered plasticizer.
<8> The method according to any one of <1> to <7>, wherein the powdered wetting agent comprises propylene glycol monostearate, a glycerol fatty acid ester, or a mixture containing the propylene glycol monostearate and the glycerol fatty acid ester as main ingredients.
<9> The method according to any one of <1> to <8>, wherein each of the powdered plasticizer and the powdered wetting agent has a volume-based average particle diameter of 1 to 500 µm, wherein the volume-based average particle diameter is determined by dry laser diffractometry.

The present invention enables the coating by spray of powdered coating ingredients, containing HPMCAS, alone, and in a single layering process alone. In addition, the present invention allows significant reduction of time required for preparing the coating ingredients and the coating process.

### DETAILED DESCRIPTION OF THE INVENTION

The present invention will be described in more detail hereunder.

The method of manufacturing a solid pharmaceutical preparation coated with a solvent-free coating material includes the following steps (A) to (C) of:
a preliminary mixing step (A) of mixing a powdered HPMCAS, a powdered plasticizer, and a powdered wetting agent in advance to prepare a precursor mixture;
a mixing step (B) of mixing the precursor mixture, and a solid pharmaceutical preparation to produce a mixture product; and
a step (C) of heating the mixture product to obtain a coated solid pharmaceutical preparation.

### Preliminary Mixing Step (A)

### <Precursor mixture>

### [HPMCAS]

The HPMCAS is a cellulose derivative which has four types of substituents: an acetyl group, a succinyl group, a methoxy group, and a hydroxypropoxy group, on cellulose, and the one in powdery form will be used in the manufacturing method of the present invention.

It is preferred that the HPMCAS contain methoxy groups in an amount of 12.0 to 28.0% by mass, more preferably 16.0 to 27.0% by mass, and even more preferably 20.0 to 26.0% by mass.

It is preferred that the HPMCAS contain hydroxypropoxy groups in an amount of 4.0 to 23.0% by mass, more preferably 4.5 to 17.0% by mass, and even more preferably 5.0 to 10.0% by mass.

It is preferred that the HPMCAS contain acetyl groups in an amount of 2.0 to 16.0% by mass, more preferably 3.0 to 15.0% by mass, and even more preferably 5.0 to 14.0% by mass.

It is preferred that the HPMCAS contain succinyl groups in an amount of 4.0 to 28% by mass, more preferably 4.0 to 23.0% by mass, and even more preferably 4.0 to 18% by mass.

As to the preferable combined amounts of the methoxy groups, hydroxypropoxy groups, acetyl groups and succinyl groups in the HPMCAS, it is preferred that the HPMCAS contain methoxy groups in an amount of 12.0 to 28.0% by mass, hydroxypropoxy groups in an amount of 4.0 to 23.0% by mass, acetyl groups in an amount of 2.0 to 16.0% by mass, and succinyl groups in an amount of 4.0 to 28% by mass. It is more preferred that the HPMCAS contain methoxy groups in an amount of 16.0 to 27.0% by mass, hydroxypropoxy groups in an amount of 4.5 to 17.0% by mass, acetyl groups in an amount of 3.0 to 15.0% by mass, and succinyl groups in an amount of 4.0 to 23.0% by mass. It is even more preferred that the HPMCAS contain methoxy groups in an amount of 20.0 to 26.0% by mass, hydroxypropoxy groups in an amount of 5.0 to 10.0% by mass, acetyl groups in an amount of 5.0 to 14.0% by mass, and succinyl groups in an amount of 4.0 to 18.0% by mass.

The amounts of methoxy, hydroxypropyl, acetyl, and succinyl groups contained in the HPMCAS can be respectively calculated by the method as set forth in the provision of "Hypromellose acetate succinate (Hydroxypropylmethylcellulose acetate succinate)" in the Official Monographs of Japanese Pharmacopoeia 18th Edition.

The manner of obtaining HPMCAS is not limited and may be the one that is manufactured by a known method or the one that is commercially available. Examples of the method for producing the HPMCAS include a method in which hydroxypropylmethylcellulose is esterified with an acetylating agent and a succinylating agent under the presence of an aliphatic carboxylic acid to produce a reaction solution, and then the resultant reaction solution is mixed with water for allowing it to be precipitated to prepare a suspension liquid that contains HPMCAS particles after which the HPMCAS in the suspension liquid is subjected to the treatments such as washing, recovering, drying and pulverization to prepare the HPMCAS.

The volume-based average particle diameter of the HPMCAS as determined by dry laser diffractometry is preferably 1 to 10 µm in terms of forming a uniform film.

### [Plasticizer]

The plasticizer used in the manufacturing method of the present invention is of powdery form.

It is preferred in terms of efficiently forming a film during the coating that the plasticizer have a melting point of 50 to 69°C, more preferably 53 to 69°C, and even more preferably 53 to 60°C.

The plasticizer has a volume-based average particle diameter, determined by dry laser diffractometry, preferably of 1 to 500 µm, more preferably of 1 to 400 µm.

Specific examples of the plasticizer include: hydrocarbons such as paraffin, microcrystalline wax, and petrolatum; beeswaxes such as beeswax and bleached beeswax; alcohols such as cetyl alcohol and stearyl alcohol; higher fatty acids such as stearic acid; higher fatty acid esters such as carnauba wax and rice wax; beef tallow, lard, cacao butter, and hardened beef tallow oil; glycerin fatty acid esters such as hardened castor oil and glycerin monostearate; Lubri Wax-101 (hydrogenated vegetable oil), Polishing Wax-101 (a mixture of carnauba wax and paraffin), Precirol (glycerin mono-, di-, and tri-mixed palmitate), sorbitan monostearate, acetylated glycerin monostearate, sucrose fatty acid esters, polyethylene glycol; and a mixture containing these as main ingredients (more than 50% by mass). Of these, it is preferred that the plasticizer be polyethylene glycol, stearic acid, or a mixture containing these as the main ingredients (more than 50% by mass) among which polyethylene glycol and stearic acid are more preferred.

One or more types of plasticizers may be used.

### [Wetting Agent]

The wetting agent used in the manufacturing method of the present invention is of powdery form.

It is preferred in terms of efficiently laminating the HPMCAS and the plasticizer onto a solid pharmaceutical preparation that the wetting agent have a melting point that is lower by at least 8°C, more preferably by at least 10°C, than the melting point of the plasticizer. The upper limit of the melting point is not particularly limited but it is preferred in terms of availability that the limit be lower than the melting point of the plasticizer and 45°C or below. The lower limit of the melting point of the wetting agent is not particularly limited but it is preferred that the limit be 30°C, more preferably 35°C.

The term "powdery" as used herein or its associated terms refers to a state of being solid at the standard temperature (20°C) and finely pulverized.

The wetting agent has a volume-based average particle diameter, determined by dry laser diffractometry, preferably of 1 to 500 µm, more preferably of 1 to 300 µm.

Specific examples of the wetting agent include, for example, poloxamer, propylene glycol stearate, and glycerin fatty acid esters. Of these, preferred are propylene glycol stearate, glycerin fatty acid esters, and a mixture thereof including these as the main ingredients (more than 50% by mass) among which propylene glycol stearate and glycerin fatty acid esters are more preferred.

One or more types of the wetting agents may be used.

Furthermore, into the coating material may be added not only the precursor mixture, containing the above-listed powdery ingredients, but also any other pharmaceutically acceptable drugs, colorants, pigments, lubricants and/or anti-adhesive agents that may be normally used in a coating material.

The drug is not particularly limited so long as it can be orally administered. Examples of such drug include a drug for the central nervous system, a drug for the cardiovascular system, a drug for the respiratory system, a drug for the digestive system, an antibiotic, an antitussive/expectorant, an antihistamine, an antipyretic/anti-inflammatory/analgesic agent, a diuretic, a sympathomimetic stimulant, an antimalarial agent, an antidiarrheal agent, a psychotropic, and vitamins and derivatives thereof.

Examples of the drug for the central nervous system include diazepam, idebenone, aspirin, ibuprofen, paracetamol, naproxen, piroxicam, diclofenac, indomethacin, sulindac, lorazepam, nitrazepam, phenytoin, acetaminophen, ethenzamide, ketoprofen and chlordiazepoxide.

Examples of the drug for the cardiovascular system include molsidomine, vinpocetine, propranolol, methyldopa, dipyridamole, furosemide, triamterene, nifedipine, atenolol, spironolactone, metoprolol, pindolol, captopril, isosorbide dinitrate, delapril hydrochloride, meclofenoxate hydrochloride, diltiazem hydrochloride, etilefrine hydrochloride, digitoxin, propranolol hydrochloride, and alprenolol hydrochloride.

Examples of the drug for the respiratory system include amlexanox, dextromethorphan, theophylline, pseudoephedrine, salbutamol, and guaifenesin.

Examples of the drug for the digestive system include a benzimidazole drug having antiulcer action, such as 2-[[3-methyl-4-(2,2,2-trifluoroethoxy)-2-pyridyl]methylsulfinyl]benzimidazole and 5-methoxy-2-[(4-methoxy-3,5-dimethyl-2-pyridyl)methylsulfinyl]benzimidazole; cimetidine; ranitidine; pirenzepine hydrochloride; pancreatin; bisacodyl; and 5-aminosalicylic acid.

Examples of the antibiotic include talampicillin hydrochloride, bacampicillin hydrochloride, cefaclor, and erythromycin.

Examples of the antitussive/expectorant include noscapine hydrochloride, carbetapentane citrate, dextromethorphan hydrobromide, isoaminile citrate, and dimemorfan phosphate.

Examples of the antihistamine include chlorpheniramine maleate, diphenhydramine hydrochloride, and promethazine hydrochloride.

Examples of the antipyretic/anti-inflammatory/analgesic agent include ibuprofen, diclofenac sodium, flufenamic acid, sulpyrine, aspirin, and ketoprofen.

Examples of the diuretic include caffeine.

Examples of the sympathomimetic stimulant include dihydrocodeine phosphate and dl-methylephedrine hydrochloride.

Examples of the antimalarial agent include quinine hydrochloride.

Examples of the antidiarrheal agent include loperamide hydrochloride.

Examples of the psychotropic include chlorpromazine.

Examples of the vitamins and derivatives thereof include vitamin A, vitamin B1, fursultiamine, vitamin B2, vitamin B6, vitamin B12, vitamin C, vitamin D, vitamin E, vitamin K, calcium pantothenate, and tranexamic acid.

Examples of the colorant include yellow iron oxide, diiron trioxide (red), orange essence, brown iron oxide, caramel, light anhydrous silicic acid, food blue No. 5, food yellow No. 4, food yellow No. 4 aluminum lake, food yellow No. 5, food red No. 2, food red No. 3, food red No. 102, food blue No. 2, talc, fluorescein sodium, green tea powder, vitamin C, food lake coloring, carotenoid-based dyes, flavonoid-based dyes, and quinone-based dyes.

Examples of the pigment include carbon black, food red No.2 aluminum lake, food red No.3 aluminum lake, food red No.40 aluminum lake, food yellow No.4 aluminum lake, food yellow No.5 aluminum lake, food blue No.1 aluminum lake, food blue No.2 aluminum lake, diiron trioxide, yellow diiron trioxide, and black iron oxide.

Examples of the lubricants include magnesium stearate, calcium stearate, colloidal silica, and stearic acid.

Examples of the anti-adhesive agents include talc.

### <Preliminary Mixing Step>

Preliminary mixing of the ingredients is preferable to uniformly blend the respective ingredients. Examples of such method include manual mixing that is performed in a condition where the respective ingredients are put in a plastic bag and batch-type mixing using a machine such as V type blender.

The time and frequency of the preliminary mixing are not limited so long as the ingredients can be uniformly mixed in advance.

Although the mixing ratio of the ingredients in the preliminary mixing step is not particularly limited, it is preferred that the amount of the powdered plasticizer be, for example, 1 to 200 parts by mass, more preferably 1 to 100 parts by mass, per 100 parts by mass of the powdered HPMCAS. It is also preferred that the amount of the powdered wetting agent be 1 to 50 parts by mass, more preferably 1 to 30 parts by mass, per 100 parts by mass of the powdered HPMCAS.

### Mixing Step (B)

### <Solid Pharmaceutical Preparation >

The solid pharmaceutical preparation may be in a form of, for example, a granule, a fine granule, a tablet, a powder, a pill, a troche, a capsule, or the active ingredient. It is preferred in terms of forming a uniform film that the solid pharmaceutical preparation have a shape close to a spherical shape.

### <Mixing Step>

The manner of mixing the precursor mixture and the solid pharmaceutical preparation is not limited, and any method may be used so long as the precursor mixture and the solid pharmaceutical preparation are uniformly mixed with each other. Specifically, a manual mixing or a batch type mixing may be used for the mixing in a manner similar to the preliminary mixing, or alternatively, the solid pharmaceutical preparation and the precursor mixture may be stirred in a coating machine to be mixed with each other. A preferable example of such mixing method includes a method of continually spraying the precursor mixture onto a solid pharmaceutical preparation. Examples of such coating machine for use in the mixing step include a centrifugal tumbling coating machine, a pan coating machine, and a fluid bed coating machine among which a centrifugal tumbling coating machine is preferred in terms of agitation.

The mixing step may be performed under heating; that is, the mixing may be performed simultaneously with the heating step to be explained below. When the mixing step is carried out under heating, the heating conditions are the same as those of the heating step to be explained below.

Although the mixing ratio of the precursor mixture and the solid pharmaceutical preparation in the mixing step is not particularly limited, it is preferred that the amount of the precursor mixture be, for example, 0.01 to 500 parts by mass, more preferably 0.1 to 300 parts by mass, per 100 parts by mass of the solid pharmaceutical preparation.

### Heating Step (C)

### <Heating Step>

The heating step allows the precursor mixture, located on the solid pharmaceutical preparation, to be melt and coat the solid pharmaceutical preparation to form a film, thus forming a coating layer on at least a part of a surface of the solid pharmaceutical preparation.

In terms of film formation, it is preferred that the product temperature of the solid pharmaceutical preparation in the heating step be set at a temperature that is higher than the melting point of the plasticizer, more preferably at a temperature of 53 to 80°C, and even more preferably at a temperature of 53 to 73°C.

It is preferred in terms of film formation that the heating time be 1 minute to 72 hours, more preferably 5 minutes to 24 hours, and even more preferably 20 to 120 minutes.

In this heating step, an apparatus used for the mixing or heating step, or any other suitable apparatus may be used, and an example of such apparatus that may be used and not mentioned above include an oven dryer.

The heating step may be carried out simultaneously with the mixing step. That is, the solid pharmaceutical preparation and the precursor mixture may be heated while being mixed with each other. Alternatively, after the mixing step is carried out while heating the same, heating may be continued while the mixing is stopped.

Furthermore, talc, silicon dioxide (SiO₂) such as Aerosil and Adsolider 101, magnesium stearate, corn starch, or any other suitable materials can be additionally sprayed for the purpose of preventing the granules from sticking together during the heating step.

As explained above, the present invention provides a coated pharmaceutical preparation (solid pharmaceutical preparation) using a solvent-free precursor mixture that does not contain any solvents at all.

In addition, steps (A) to (C) may be repeated using different compositions in the precursor mixture to form a multi-layered coating film.

Furthermore, the solid pharmaceutical preparation obtained using the manufacturing method according to the present invention may be further coated with any other polymer compound(s).

### WORKING EXAMPLES

The present invention is described in greater detail hereunder with reference to the working and comparative examples, and the invention shall not be limited to these working examples.

The physical properties were measured in accordance with the methods as explained above unless otherwise stated. Moreover, the sign "%" as used hereunder for describing the amounts of substituents refers to "% by mass".

### <Test 1: Preparation of Theophylline-containing Granules (Solid pharmaceutical preparation) >

450 g of granule (SmartEx^{®} QD-100 manufactured by Shin-Etsu Chemical Co., Ltd.) consisting of D-mannitol, low-substituted hydroxypropyl cellulose and polyvinyl alcohol (fully hydrolyzed), 25 g of theophylline, 10 g of low-substituted hydroxypropyl cellulose (having 11.1% of hydroxypropoxy groups and average particle diameter of 20.2 µm), and 15 g of polyvinyl alcohol (PE-05JPS manufactured by JAPAN VAM & POVAL CO.,LTD) were put in a wet granulation machine (GRANUMEIST^{®} manufactured by FREUND Corp.) and agitated for 5 minutes while adding 140 g of water to form granules. The granules were put into an extrusion granulation machine (Dome Granulator manufactured by DALTON Corporation) and extruded through a dome die having φ 1mm sieve mesh size. The extruded products were put into a spheronizer (Marumerizer^{™} manufactured by DALTON Corporation) to shape the granule products to have spherical shapes from cylindrical shapes, and the resultant granules were left to stand and dried for 24 hours using an air blow oven at 50°C to obtain a solid pharmaceutical preparation of spherical core granules.

### <Working Example 1>

500 g of HPMCAS (having 22.3% of methoxy groups, 6.9% of hydroxypropoxy groups, 7.7% of acetyl groups, 15.1% of succinyl groups; average particle diameter of 4.6 µm), 250 g of polyethylene glycol (Macrogol 4000PS, manufactured by NOF Corp., listed as PEG 4000 in Table 1; same below), 100 g of propylene glycol monostearate (manufactured by Kao Homotex PS-200V, manufactured by Kao Corp.), and 300 g of talc were put in a plastic bag and mixed by hand for 100 times in advance to prepare a precursor mixture.

Next, 500 g of the solid pharmaceutical preparation prepared in Test 1 was put in a centrifugal tumbling coating machine (CF granulator CF-360, manufactured by Freund Corporation). The precursor mixture was then continuously added from the powder supply part at the top of the machine at a rate of 11.5 g/min while performing centrifugal fluidization at an inlet air temperature of 90°C and a product temperature of 68°C to mix the solid pharmaceutical preparation and the precursor mixture. After performing the mixing step, 2.5 g of silicon dioxide (Adsolider^{®} 101 manufactured by Freund Corp.) was added to the granules and a heating step was continued while performing centrifugal fluidization for 30 minutes without changing the inlet air temperature. As the result, a solid pharmaceutical preparation coated with a solvent-free coating material was obtained. The coating yield (hereinafter referred to as the yield) was 96.40%. The yield was calculated using the following formula. Yield (%) = [(Weight of the recovered coated solid pharmaceutical preparation - Weight of the solid pharmaceutical preparation before being coated)/(Supplied weight of the coating composition)] × 100

### <Working Example 2>

350 g of HPMCAS (having 22.3% of methoxy groups, 6.9% of hydroxypropoxy groups, 7.7% of acetyl groups, 15.1% of succinyl groups; average particle diameter of 4.6 µm), 175 g of stearic acid (Kolliwax^{®} S Fine, manufactured by BASF Corporation), 70 g of propylene glycol monostearate (Kao Homotex PS-200V, manufactured by Kao Corp.), and 210 g of talc were put in a plastic bag and mixed by hand for 100 times in advance to prepare a precursor mixture.

Next, 500 g of the solid pharmaceutical preparation prepared in Test 1 was put in a centrifugal fluidizing coating machine (CF granulator CF-360, manufactured by Freund Corporation). The precursor mixture was then continuously added from the powder supply part at the top of the machine at a rate of 11.5 g/min while performing centrifugal fluidization at an inlet air temperature of 90°C and a product temperature of 68°C to mix the solid pharmaceutical preparation and the precursor mixture. After performing the mixing step, 2.5 g of silicon dioxide (Adsolider^{®} 101 manufactured by Freund Corp.) was added to the granules and a heating step was continued while performing centrifugal fluidization for 30 minutes without changing the inlet air temperature. As the result, a solid pharmaceutical preparation coated with a solvent-free coating material was obtained. The yield was calculated in a manner same as the working example 1, and it turned out that the yield was 94.45 %.

### <Working Example 3>

350 g of HPMCAS (having 22.3% of methoxy groups, 6.9% of hydroxypropoxy groups, 7.7% of acetyl groups, 15.1% of succinyl groups; average particle diameter of 4.6 µm), 157.5 g of polyethylene glycol (Macrogol 4000PS, manufactured by NOF Corp.), 87.5 g of stearic acid (Kolliwax^{®} S Fine, manufactured by BASF Corporation), 70 g of propylene glycol monostearate (Kao Homotex PS-200V, manufactured by Kao Corp.), and 210 g of talc were put in a plastic bag and mixed by hand for 100 times in advance to prepare a precursor mixture.

Next, 500 g of the solid pharmaceutical preparation prepared in Test 1 was put in a centrifugal fluidizing coating machine (CF granulator CF-360, manufactured by Freund Corporation). The precursor mixture was then continuously added from the powder supply part at the top of the machine at a rate of 11.5 g/min while performing centrifugal fluidization at an inlet air temperature of 90°C and a product temperature of 68°C to mix the solid pharmaceutical preparation and the precursor mixture. After performing the mixing step, 2.5 g of silicon dioxide (Adsolider^{®} 101 manufactured by Freund Corp.) was added to the granules and a heating step was continued while performing centrifugal fluidization for 30 minutes without changing the inlet air temperature. As the result, a solid pharmaceutical preparation coated with a solvent-free coating material was obtained. The yield was calculated in a manner same as the working example 1, and it turned out that the yield was 96.36%.

### <Working Example 4>

350 g of HPMCAS (having 22.3% of methoxy groups, 6.9% of hydroxypropoxy groups, 7.7% of acetyl groups, 15.1% of succinyl groups; average particle diameter of 4.6 µm), 157.5 g of polyethylene glycol (Macrogol 6000SP, manufactured by Sanyo Chemical Industries, Ltd., listed as PEG 6000 in Table 1), 87.5 g of stearic acid (Kolliwax^{®} S Fine, manufactured by BASF Corporation), 70 g of propylene glycol monostearate (Kao Homotex PS-200V, Kao Corp.), and 210 g of talc were put in a plastic bag and mixed by hand for 100 times in advance to prepare a precursor mixture.

Next, 500 g of the solid pharmaceutical preparation prepared in Test 1 was put in a centrifugal fluidizing coating machine (CF granulator CF-360, manufactured by Freund Corporation). The precursor mixture was then continuously added from the powder supply part at the top of the machine at a rate of 11.5 g/min while performing centrifugal fluidization at an inlet air temperature of 90°C and a product temperature of 68°C to mix the solid pharmaceutical preparation and the precursor mixture. After performing the mixing step, 2.5 g of silicon dioxide (Adsolider^{®} 101 manufactured by Freund Corp.) was added to the granules and a heating step was continued while performing centrifugal fluidization for 30 minutes without changing the inlet air temperature. As the result, a solid pharmaceutical preparation coated with a solvent-free coating material was obtained. The yield was calculated in a manner same as the working example 1, and it turned out that the yield was 96.35%.

### <Working Example 5>

350 g of HPMCAS (having 22.3% of methoxy groups, 6.9% of hydroxypropoxy groups, 7.7% of acetyl groups, 15.1% of succinyl groups; average particle diameter of 4.6 µm), 157.5 g of polyethylene glycol (Macrogol 4000PS, manufactured by NOF Corp.), 87.5 g of stearic acid (Kolliwax^{®} S Fine, manufactured by BASF Corporation), 70 g of glycerin fatty acid ester (POEM W-90VP manufactured by Riken Vitamin Co., Ltd.), and 210 g of talc were put in a plastic bag and mixed by hand for 100 times in advance to prepare a precursor mixture.

Next, 500 g of the solid pharmaceutical preparation prepared in Test 1 was put in a centrifugal fluidizing coating machine (CF granulator CF-360, manufactured by Freund Corporation). The precursor mixture was then continuously added from the powder supply part at the top of the machine at a rate of 11.5 g/min while performing centrifugal fluidization at an inlet air temperature of 90°C and a product temperature of 68°C to mix the solid pharmaceutical preparation and the precursor mixture. After performing the mixing step, 2.5 g of silicon dioxide (Adsolider^{®} 101 manufactured by Freund Corp.) was added to the granules and a heating step was continued while performing centrifugal fluidization for 30 minutes without changing the inlet air temperature. As the result, a solid pharmaceutical preparation coated with a solvent-free coating material was obtained. The yield was calculated in a manner same as the working example 1, and it turned out that the yield was 94.50%.

### <Working Example 6>

500 g of HPMCAS (having 22.3% of methoxy groups, 6.9% of hydroxypropoxy groups, 7.7% of acetyl groups, 15.1% of succinyl groups; average particle diameter of 4.6 µm), 250 g of polyethylene glycol (Macrogol 4000PS, manufactured by NOF Corp., listed as PEG 4000 in Table 1; same below), 75 g of propylene glycol monostearate (Kao Homotex PS-200V, manufactured by Kao Corp.), and 300 g of talc were put in a plastic bag and mixed by hand for 100 times in advance to prepare a precursor mixture.

Next, 500 g of the solid pharmaceutical preparation prepared in Test 1 was put in a centrifugal fluidizing coating machine (CF granulator CF-360, manufactured by Freund Corporation). The precursor mixture was then continuously added from the powder supply part at the top of the machine at a rate of 11.3 g/min while performing centrifugal fluidization at an inlet air temperature of 90°C and a product temperature of 68°C to mix the solid pharmaceutical preparation and the precursor mixture. After performing the mixing step, 2.5 g of silicon dioxide (Adsolider^{®} 101 manufactured by Freund Corp.) was added to the granules and a heating step was continued while performing centrifugal fluidization for 30 minutes without changing the inlet air temperature. As the result, a solid pharmaceutical preparation coated with a solvent-free coating material was obtained. The coating yield (herein referred to as the yield) was 76.40%.

### <Comparative Example 1>

500 g of HPMCAS (having 22.3% of methoxy groups, 6.9% of hydroxypropoxy groups, 7.7% of acetyl groups, 15.1% of succinyl groups; average particle diameter of 4.6 µm), 250 g of polyethylene glycol (Macrogol 4000PS, manufactured by NOF Corp.), 300 g of talc were put in a plastic bag and mixed by hand for 100 times in advance to prepare a precursor mixture.

Next, 500 g of the solid pharmaceutical preparation prepared in Test 1 was put in a centrifugal fluidizing coating machine (CF granulator CF-360, manufactured by Freund Corporation). The precursor mixture was then continuously added from the powder supply part at the top of the machine at a rate of 11.5 g/min while performing centrifugal fluidization at an inlet air temperature of 90°C and a product temperature of 68°C to mix the solid pharmaceutical preparation and the precursor mixture. After performing the mixing step, 2.5 g of silicon dioxide (Adsolider^{®} 101 manufactured by Freund Corp.) was added to the granules and a heating step was continued while performing centrifugal fluidization for 30 minutes without changing the inlet air temperature. As the result, a solid pharmaceutical preparation coated with a solvent-free coating material was obtained. The yield was calculated in a manner same as the working example 1, and it turned out that the yield was 8.2%.

**Table 1**

| | HPMCAS | | Plasticizer (1) | | | | Plasticizer (2) | | | | Wetting Agent | | | | Melting point difference | |
|---|---|---|---|---|---|---|---|---|---|---|---|---|---|---|---|---|
| | Additive Amount (g) | Average Particle Diameter (µm) | Name | Melting Point (°C) | Average Particle Diameter (µm) | Additive Amount (g) | Name | Melting Point (°C) | Average Particle Diameter (µm) | Additive Amount (g) | Name | Melting Point (°C) | Average Particle Diameter (µm) | Additive Amount (g) | Plasticizer(1) -Wetting Agent (°C) | Plasticizer(2) -Wetting Agent (°C) |
| Working Example 1 | 500 | 4.7 | PEG 4000 | 55.4 | 368 | 250 | - | - | - | - | Propylene glycol monostearate | 43.1 | 255 | 100 | 12.3 | - |
| Working Example 2 | 350 | 4.7 | Stearic acid | 56.0 | 114 | 175 | - | - | - | - | Propylene glycol monostearate | 43.1 | 255 | 70 | 12.9 | - |
| Working Example 3 | 350 | 4.7 | PEG 4000 | 55.4 | 368 | 157.5 | Stearic acid | 56.0 | 114 | 87.5 | Propylene glycol monostearate | 43.1 | 255 | 70 | 12.3 | 12.9 |
| Working Example 4 | 350 | 4.7 | PEG 6000 | 58.5 | 314 | 157.5 | Stearic acid | 56.0 | 114 | 87.5 | Propylene glycol monostearate | 43.1 | 255 | 70 | 15.4 | 12.9 |
| Working Example 5 | 350 | 4.7 | PEG 4000 | 55.4 | 368 | 157.5 | Stearic acid | 56.0 | 114 | 87.5 | Glycerin fatty acid ester | 40.6 | 116 | 70 | 14.8 | 15.4 |
| Working Example 6 | 500 | 4.7 | PEG 4000 | 55.4 | 368 | 250 | - | - | - | - | Propylene glycol monostearate | 43.1 | 255 | 75 | 12.3 | - |
| Comparative Example 1 | 500 | 4.7 | PEG 4000 | 55.4 | 368 | 250 | - | - | - | - | - | - | - | - | - | - |

**Table 2**

| | Precursor mixture (g) | Solid pharmaceutical preparation (g) | Coating yield (%) |
|---|---|---|---|
| Working Example 1 | 1150 | 500 | 96.40 |
| Working Example 2 | 805 | 500 | 94.45 |
| Working Example 3 | 875 | 500 | 96.36 |
| Working Example 4 | 875 | 500 | 96.35 |
| Working Example 5 | 875 | 500 | 94.50 |
| Working Example 6 | 1125 | 500 | 76.40 |
| Comparative Example 1 | 1050 | 500 | 8.2 |

## Claims

1. A method of manufacturing a solid pharmaceutical preparation coated with a solvent-free coating material comprising the steps of:
mixing a powdered hydroxypropylmethylcellulose acetate succinate, a powdered plasticizer, and a powdered wetting agent in advance, as a preliminary mixing step, to prepare a precursor mixture;
mixing the precursor mixture and a solid pharmaceutical preparation, as a mixing step, to produce a mixture product; and
heating the mixture product, as a heating step, to obtain a coated solid pharmaceutical preparation.

2. The method according to claim 1, wherein the mixing step is performed while heating.

3. The method according to claim 1 or 2, wherein the mixing step comprises continually spraying the precursor mixture onto the solid pharmaceutical preparation to produce a mixture product.

4. The method according to claim 1 or 2, wherein the powdered hydroxypropylmethylcellulose acetate succinate has a volume-based average particle diameter of 1 to 10 µm, wherein the volume-based average particle diameter is determined by dry laser diffractometry.

5. The method according to claim 1 or 2, wherein the powdered plasticizer has a melting point of 50 to 69°C.

6. The method according to claim 1 or 2, wherein the powdered plasticizer is at least one material selected from polyethylene glycol and stearic acid.

7. The method according to claim 1 or 2, wherein the powdered wetting agent has a melting point at least 8°C lower than the melting point of the powdered plasticizer.

8. The method according to claim 1 or 2, wherein the powdered wetting agent comprises propylene glycol monostearate, a glycerol fatty acid ester, or a mixture containing the propylene glycol monostearate and the glycerol fatty acid ester as main ingredients.

9. The method according to claim 1 or 2, wherein each of the powdered plasticizer and the powdered wetting agent has a volume-based average particle diameter of 1 to 500 µm, wherein the volume-based average particle diameter is determined by dry laser diffractometry.
